# EUROPEAN PATENT APPLICATION

(11) **EP 3 708 171 A1**
(43) Date of publication of application: **16.09.2020**
(21) Application number: 19161915.4
(22) Date of filing: 11.03.2019
(51) Int. Cl.: A61K 31/593, A61P 3/02

(54) **TREATMENT OF VITAMIN D DEFICIENCY WITH 25(OH) VITAMIN D IN CRITICALLY ILL PATIENTS**

(71) Applicant: Bouillon, Roger, 3020 Herent (BE)
(72) Inventor: Bouillon, Roger, 3020 Herent (BE)
(74) Representative: De Clercq & Partners

(57) **Abstract**

The invention provides the treatment of vitamin D deficiency with high concentration of 25OHD to restore said vitamin D deficiency.

## Description

### FIELD OF THE INVENTION

The present invention relates generally to methods and compositions for the treatment or prevention of vitamin D deficiency in a patient, particularly in a critically ill patient.

### BACKGROUND

Vitamin D is a precursor of a steroid hormone that has been shown to be involved in different processes. It is best known as the agent that is essential for intestinal calcium absorption and mineral deposition in bone matrix. Indeed, the absence of vitamin D itself, or absence of the essential enzymes responsible for its metabolic activation into 1a,25-dihydroxyvitamin D, or the absence of its receptor, results in a serious disease known as rickets (in infants and children before the closure of the growth plate) or osteomalacia (in adults). Moreover, the vitamin D endocrine system (vitamin D, the enzymes involved in its metabolism, its transport in serum and inside the cell, the vitamin D receptor or receptors and the genes and proteins regulated by this system) probably also has many extra-skeletal effects such as a role in immunity, endothelial and mucosal function, muscle function, respiratory function, and the regulation of the cell cycle.

Vitamin D deficiency has been associated with a number of diseases including heart disease and some forms of cancer, but also bone pain, muscle weakness and falls, increased blood pressure and cardiovascular risk factors and cardiovascular endpoints, infections (especially upper respiratory infections), autoimmune diseases, metabolic diseases including diabetes, and depression. In older patients, vitamin D deficiency has been linked with an increased risk for developing some forms of dementia, including Alzheimer's disease. Finally, a poor vitamin D status has been associated with increased mortality risks as well in the general population (US, Europe, Japan..) and intervention studies revealed a modest but significant beneficial effects in reducing mortality risk during the follow-up period of these studies (Bouillon et al. 2018, Endo Rev, doi: 10.1210/er.2018-00126).

Patients admitted to hospitals because of severe trauma (e.g. major surgery), such as patients requiring ICU care are frequently (severely) vitamin D deficient. This may be due to a poor vitamin D status before acute illness for a variety of reasons; an increased distribution volume of DBP/25OHD or both; sequestration of vitamin D in fat or other tissues; etc: no valid mechanism has been truly proven to be the main driver of this vitamin D deficiency in such patients.

In particular, critically ill patients have been found to have low circulating 25-hydroxyvitamin D [25(OH)vitamin D or 25OHD], vitamin D binding protein (DBP) and 1,25-dihydroxyvitamin D [1,25(OH)2D]. While this has been found to be correlated with adverse clinical outcome (higher morbidity and higher mortality rates), it is not clear whether this association represents causality, or whether a low vitamin D status merely reflects the severity of illness (reverse causality).

Only a few studies have investigated the possibility of restoring the deficient vitamin D status in critically ill patients. Ingels et al. (2010, Critical Care 2010, 14(Suppl 1):P590 (doi: 10.1186/cc8822) describe that the daily administration of 15 µg 25(OH)D increased the serum levels of 25OHD, but caused only a transient increase in level of 1,25(OH)2D3. The oral administration of a much high dose of vitamin D3 also did not affect outcome of critically ill patients, except for a subgroup with baseline severe vitamin D deficiency, for whom a lower hospital mortality was observed (Amrein et al., 2018, Endocr Connect. 1;7(12):R304-R315. doi: 10.1530/EC-18-0184; Langlois et al., 2018, Clin Nutr. 3(4):1238-1246. doi: 10.1016/j.clnu.2017.05.006).

Improving the vitamin D status, particularly in such critically ill patients may have beneficial effects on bone, muscle, the immune system, the respiratory system and/or metabolism. There thus remains a need for methods for treating vitamin D deficiency, more particularly in the rapid correction of vitamin D deficiency in critically ill patients, as such patients have many morbidity problems (and increased mortality risks) that are associated with vitamin D deficiency.

### SUMMARY OF THE INVENTION

The present invention provides compositions and methods for the treatment of vitamin D deficiency in a subject, particularly in a critically ill patient. Stated differently, the present invention provides methods for effectively, safely and rapidly restoring serum 25OHD levels to at least 20 ng/ml 25OHD or at least 30 ng/ml 25OHD) in vitamin D deficient subjects and maintaining blood 25OHD levels at such optimal levels, comprising parenterally, particularly intravenously, administering to a subject, particularly a critically ill subject, a 25OHD formulation, as well as pharmaceutical compositions or formulations comprising 25OHD for use in such methods.

In particular embodiments the invention provides pharmaceutical compositions comprising 25OHD for use in the treatment of vitamin D deficiency in a critically ill patient, wherein said treatment comprises administering the equivalent of a daily dosage of at least 25 µg 25OHD/day to said patient over a period of at least two days. In particular embodiments, the treatment comprises administering a daily dosage of at least 25µg/day to said patient for at least two days. In further embodiments, said administration is intravenous or oral.

In particular embodiments, the methods comprise administering a bolus of 25OHD on day 0 prior to administering a daily dosage of at least 25 µg 25OHD/day as of day 1, for at least 2 consecutive days. In further particular embodiments, the bolus of 25OHD is at least 200µg. In particular embodiments, the bolus is determined based on the distribution volume and the target serum concentration. Additionally or alternatively, the starting 25OHD serum concentration of the patient is determined prior to treatment and the daily dosage is determined based on the difference between a target concentration and said starting concentration.

In particular embodiments, the treatment comprises administering the equivalent of the daily dosage of at least 25µg 25OHD/day until the level of serum 25OHD in a plasma sample of said patient is restored to at least 20 ng/ml or to at least 30 ng/ml. In further particular embodiments, the treatment comprises administering the equivalent of a daily dosage of at least 25 µg 25OHD/day for at least 5 or at least 7 consecutive days to said patient. In further particular embodiments, the treatment comprises, determining the serum 25OHD level of the patient after two days, and adjusting the equivalent of the daily dosage of said 25OHD accordingly.

The invention further provides pharmaceutical compositions comprising between 25 and 60 µg 25OHD and compositions comprising between 25 and 60µg/ml 25OHD. Additionally, the invention provides kits comprising a pharmaceutical composition comprising a first vial comprising between 25 and 60 µg/ml 25OHD in ethanol and a second vial comprising between 200-300µg/ml of 25OHD.

The invention further provides methods for determining the bolus dosage for administering to a patient or patient group for treating vitamin D deficiency, said methods comprising (a) determining the serum 25OHD concentration of said patient or patient group, (b) determining the target 25OHD concentration of said patient or patient group, and (c) determining the bolus dosage for administration to said patient or patient group based on the distribution volume in said patient. In particular embodiments, the patient is a critically ill patient and said distribution volume is 18 L.

The invention further provides methods for determining the optimal replacement dosage for administering to a critically ill patient, the method comprising, determining the baseline serum concentration of 25OHD of a patient, determining the target value of 25OHD level for the patient, deducing therefrom the increase required and then calculating the amount of 25OHD that needs to be administered daily in order to ensure the desired increase, based on the increase ensured by 1µg/ml 25OHD in a comparable patient group.

### BRIEF DESCRIPTION OF THE FIGURES

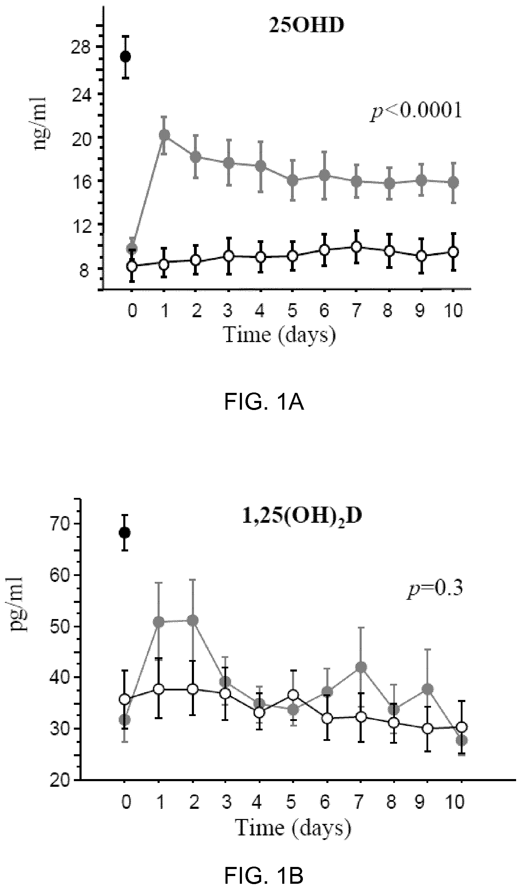

Figure 1 shows the effect of IV 25OHD supplementation on serum concentrations of 25OHD (Fig 1A) and 1,25(OH)2D (Fig 1b). Healthy controls, 25OHD and placebo treated patients are represented as black, grey and white dots, respectively.

### DETAILED DESCRIPTION

The present invention will be described with respect to particular embodiments but the invention is not limited thereto but only by the claims. Any reference signs in the claims shall not be construed as limiting the scope thereof.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" when referring to recited members, elements or method steps also include embodiments which "consist of" said recited members, elements or method steps.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

The recitation of numerical ranges by endpoints includes all numbers and fractions subsumed within the respective ranges, as well as the recited endpoints.

All documents cited in the present specification are hereby incorporated by reference in their entirety.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In its broadest sense, the term a "critically ill patient", as used herein refers to a patient who has sustained or is at risk of sustaining acutely life-threatening single or multiple organ system failure due to disease or injury, a patient who is being operated and where complications supervene, and a patient who has been operated in a vital organ within the last week or has been subject to major surgery within the last week. For instance, the acutely life-threatening organ system failure may be induced by an injury, a trauma, a medical treatment, an infection, a stroke, vascular or coronary bypass surgery, shock, preferably cardiogenic, hemorrhagic or septic shock, or a treatment of myocardial infarction. In particular, the term a "critically ill patient", as used herein refers to a patient who has sustained or is at risk of sustaining acutely life-threatening single or multiple organ system failure due to disease or injury, or a patient who is being operated and where complications supervene. In an even more particular sense, the term a "critically ill patient", as used herein refers to a patient who has sustained or is at risk of sustaining acutely life-threatening single or multiple organ system failure due to disease or injury. Similarly, these definitions apply to similar expressions such as "critical illness in a patient" and a "patient is critically ill". A critically ill patient is typically treated in a specifically adapted part of a hospital or clinic, referred to as an "Intensive Care Unit" (herein designated ICU).

The term "administration" as used herein refers to delivery of at least one therapeutic agent to a patient. The term "parenterally" or "parenteral administration" as used herein means administration of a product by means of injection, such as injection into a vein (intravenous administration). The present invention also envisages other forms of administration such as oral administration.

The term "replacement dosage" as used herein refers to a dosage administered on a regular basis, i.e. et at least twice over a period of at least two days, and preferably at least 10 days and is intended, in the context of the present invention, to systematically replace the deficit of 25OHD in the serum level of the patient. The term "daily dosage" as used herein refers to a dosage to be administered daily, for at least two consecutive days. Unless otherwise specified, the daily dosage is administered as of the start of the treatment, typically referred to as day 0 or day 1. In particular embodiments, the treatment can involve an administration on day 0, which is one day (typically 12-36hrs, preferably about 24hrs) before the start of the administration of the daily dosage. In the context of the present invention, the aim of the replacement dosage is to ensure that the deficit in serum 25OHD is compensated such that the desired 25OHD level is maintained. It will be understood to the skilled person that, in view of the extended half-life of 25OHD in particular embodiments, a daily dosage can be replaced by administering e.g. double the daily dosage every two days, three times the daily dosage every three days or even 7 times the daily dosage every 7 days. Thus, where reference is made to "an equivalent of a daily dosage of 25µg/day" this implies the administration of either at least 25µg/day, or at least 50µg every two days, at least 75µg every three days, etc.

The term "bolus" as used herein refers to a one-time administration of a dosage which is significantly higher than the daily dosage. Typically, a bolus is administered before starting the administration of a daily dosage regimen, more particularly in the context of the present invention, the administration of the bolus is performed the day before starting the daily dosage regimen.

As used herein, the term "25OHD" stands for 25-hydroxyvitamin D; the term encompasses both 25OHD₂ and 25OHD₃. However, the reference to 25OHD as used herein also encompasses situations where only 25OHD₃ is used or measured. For administration as used herein, typically 25OHD₃ is used, though it is also possible to envisaged the use of 25OHD₂ or combinations of 25OHD₂ and 25OHD₃. The term "DBP" refers to vitamin D binding protein (DBP); the term "1,25(OH)2D" refers to 1,25-dihydroxyvitamin D.

The application refers to a "starting" or "baseline" concentration of 25OHD and thereby refers to the serum concentration of 25OHD before the start of the treatment described herein. Typically, the 25OHD concentration is measured in the serum of the patient before the start of treatment. The measurement can be performed 0-48 hours before the first administration as described herein. Alternatively, the starting concentration can be assumed based on an average of a patient group with comparable symptoms. For instance, in critically ill patients, the 25OHD concentration is typically between 4ng/ml and 10 ng/ml.

The application refers to "target concentration of 25OHD" to refer to the serum concentration of 25OHD which is desirable for the patient, in order to ensure proper functioning of all functions which require vitamin D. The term "vitamin D deficient" or "vitamin D deficiency" refers to a condition of the patient wherein the vitamin D status or the serum level of 25OHD is considered too low to ensure normal functioning of vitamin D-dependent functions in the body.

Most guidelines from governmental organizations such as IOM, EFSA, Nordisk and DACH countries, Australia-New Zealand, define vitamin D deficiency as serum 25OHD below 20 ng/ml (or 50 nmol/L). Others such as the (American) Endocrine society and some scientific organizations claim that serum 25OHD concentrations between 50 and 75 nmol/I represents "vitamin D insufficiency" and define therefore the optimal concentration of 25OHD as > 75 nmol/I or 30 ng/ml (Bouillon R., 2017, Nat Rev. Endocrinol 13(8):466-479). The present patent application defines a strategy to correct vitamin D deficiency as to reach serum 25OHD above at least 20 ng/ml, optionally above 30 ng/ml.

Based on a study aiming to increase and/or restore the vitamin D status in patients in need thereof, in particular in critically ill patients, the inventors have surprisingly established that under some conditions, the catabolism of 25OHD becomes at least 3-fold higher than in normal subjects of the same age. It is found that this can be addressed by administering a much higher, supra-physiological dosage of 25OHD to said patients. Optimally, in a critically ill patient, this is provided intravenously to a critically ill patient. Alternatively in critically ill patients able to be fed orally and having a well-functioning intestinal absorption, oral 25OHD may be an alternative solution. Indeed, the intestinal absorption of (water soluble) 25OHD is much better than that of (fat soluble) vitamin D itself in normal subjects as well as in patients with a variety of gastrointestinal diseases. Accordingly, the vitamin D deficiency is treated with a much higher dosage than one would reasonably consider, particularly as high dosages of the parent compound (vitamin D) are now known to have important contraindications (Gallager et al. 2016, Nat. Rev. Endocrinol., 12(11):680-684; Sanders and Seibel 2016, Nat. Rev. Endocrinol. 12(4):190-1). It is envisaged that the present invention similarly applies more generally to hospitalized patients, or other patients with a disease condition which are determined to have a vitamin D deficiency, more particularly which are unresponsive to standard vitamin D supplementation.

Accordingly, one aspect of the present invention relates to a pharmaceutical composition for parenteral administration comprising 25OHD for use in the treatment of vitamin D deficiency in a patient, wherein said treatment comprises administering a dosage of at least 25 µg 25OHD/day, for at least 2 consecutive days to said patient.

The recommended daily intake of vitamin D is in normal subjects (Institute of Medicine and most other guidelines) is 15 or 20 µg/d of vitamin D as such for adults and elderly (> 75 years of age, respectively). It is well established that the mean conversion of vitamin D into 25OHD is 1 molecule of 25OHD generated by 3 molecules of vitamin D (based on 9 RCTs comparing the relative efficacy of oral vitamin D with oral 25OHD - Quesada and Bouillon, 2018, Osteoporosis Int. 29(8):1697-1711. doi: 10.1007/s00198-018-4520-y).

Therefore, the recommended daily intake to assure a serum concentration of 20 ng/ml in 97.5% of normal subjects is 15 µg of vitamin D or 5 µg of 25OHD (and 20µg or 7µg, respectively in elderly subjects). The present invention demonstrates that the requirements of 25OHD to normalize serum 25OHD in vitamin D deficient patients, and more particularly in critically ill patients is much higher than predicted based on data in normal subjects.

The methods of the invention are of particular use in the treatment of critically ill patients. It has been established herein that patients that in critically ill patients, require increased amounts of 25OHD in order to stabilize their vitamin D level. It has been demonstrated that the expected normal replacement dose of 5 ug of 25OHD for adults is far too low for patients with acute critical illness. In a previous study a daily replacement dose of 15 ug of 25OHD (= 3 times higher than expected for normal subjects) was used and it was found that even this daily dose was insufficient to maintain serum 25OHD above the target range of 20 ng/ml aimed for in that study (Ingels et al. 2010, Critical Care 14(Suppl 1):P590 (doi: 10.1186/cc8822)). Indeed, whereas in normal subjects a daily dose of 1 ug of 25OHD increases serum 25OHD by 1.2 ng/ml (Quesada and Bouillon, Osteoporosis International 2018), a daily dose of 1 µg of 25OHD increased serum 25OHD by only 0.45 ng/ml. Accordingly, exceptionally high dosages of 25OHD are warranted. Given that these patients appear to break down 25OHD at a much higher rate than normal patients, such high levels of 25OHD will not have the same side effects as have been observed with the administration of high levels of vitamin D. Stated differently, a first aspect of the present invention relates to a method of treating a vitamin D deficiency in a patient in need thereof, particularly a critically ill patient, comprising administering to the patient, particularly the critically ill patient, a dosage of at least 25 µg 25OHD/day for at least 2 consecutive days.

A suitable daily dosage can be calculated as follows. Typically it will involve the starting or base serum concentration of 25OHD of the patient and the target concentration of 25OHD. Where it is the aim to achieve a target serum 25OHD concentrations of at least 20 ng/ml, and one starts with a mean serum 25OHD concentration of 8 ng/ml, knowing that the daily iv dose of 1 ug of 25OHD increases serum 25OHD by 0.45 ng/ml, it can be calculated that a daily replacement dosage of (20-8): 0.45 = 27 µg is advisable. Starting with a low serum 25OHD concentration of 4 ng/ml and aiming for at target of 20 ng/ml, one needs a daily replacement dose of (20-4): 0.45 = 36 µg. Further, when aiming to achieve a target serum 25OHD concentration of at least 30 ng/ml, this also affects the daily dosage requirement. When starting with a mean serum 25OHD concentration of 8 ng/ml and aiming for 30 ng/ml or higher, knowing that the daily iv dose of 1 µg of 25OHD increases serum 25OHD by 0.45 ng/ml, the daily replacement of (30-8) : 0.45 = 49 ug/d. Starting with a low serum 25OHD concentration of 4 ng/ml and aiming for at target of 30 ng/ml one needs a daily replacement dose of (30-4): 0.45 = 58 ug.

As detailed herein, it has been established that in critically ill patients it will typically be necessary to use a dosage of at least 25µg/day. Thus, where measurement of the baseline or starting value is not possible, a dosage of between 25µg/day and 30µg/day is advisable, which can be increased in case upon measurement, the 25OHD concentrations are established not to reach the target concentration.

These doses are several fold (> 5 to 10 times) higher than a skilled person could have predicted based on the knowledge before the present invention.

Thus, as detailed above, the invention provides methods for determining the optimal daily replacement dosage of a patient, taking into account the metabolism of 25OHD in critically ill patients. The methods of the invention start from the baseline serum concentration of 25OHD of a patient, the target value of 25OHD level for the patient, determines the increase required and then determines the amount of 25OHD that needs to be administered in order to ensure the desired increase, based on the increase ensured by 1µg/ml 25OHD in a comparable patient group. As detailed herein, it has been established that in critically ill patients, 1µg/ml of 25OHD can ensure a mean increase of 0.45ng/ml. This value can be used to determine the amount of 25OHD that needs to be administered in order to ensure the desired increase in a critically ill patient. The baseline value of the serum level of 25OHD of a critically ill patient can be determined by methods known in the art. In particular embodiments, the methods of the invention are used to adjust the dosage of 25OHD to be administered. In these methods, the level of 25OHD is measured and where the level of 25OHD does not correspond to the target level, the difference between the actual level and the target level of 25OHD in the serum is used to determine the increase in dosage required, taking into account that 1µg/ml of 25OHD can ensure an increase of 0.45 ng/ml.

In order to ensure that the 25OHD level can be restored quickly, the method typically involves administering a loading dose of 25OHD to be given in a single bolus. Where the situation is such that the baseline or starting concentration of 25OHD in a patient cannot be determined, a loading dose of at least 200µg should be used. It has been established that this is a minimal dose for critically ill patients. In particular embodiments, the optimal loading dose is calculated based on the distribution volume of the patient and the desired target concentration. Indeed, the present inventors have, for the first time, identified the relevance of the distribution volume for determining the optimal bolus dosage in a critically ill patient. Taking into account the distribution volume allows for an improved ability of the treatment to ensure the target concentration. Accordingly, a further aspect of the invention provides methods for determining the bolus dosage for administering to a patient for treating vitamin D deficiency, said method comprising (a) determining the serum 25OHD concentration of said patient; (b) determining the target 25OHD concentration of said patient and (c) determining the bolus dosage for administration based on the distribution volume of 25OHD in said patient. As detailed above, the serum 25OHD concentration may be measured in different ways known in the art. With regard to the target 25OHD concentration, it is also detailed herein that this can differ depending on the situation of the patient, the treating doctor, etc. In particular embodiments, the target concentration is at least 20 ng/ml 25OHD. The distribution volume of a patient is typically based on a statistical value obtained from patients with a similar profile. For instance, it has been established in the present study that the mean distribution volume for a critically ill adult can be taken as 18 L. Accordingly, in particular embodiments of this aspect of the invention, the patient is a critically ill patient and said distribution volume is 18 L. Methods of determining the distribution volume of 25OHD of a patient group are described in the examples herein and are based on analysis of administered 25OHD and the observed concentration increase in the serum.

For instance, it has been established herein that for a normal weight adult, the distribution volume of 25OHD is about 18L. In the context of the present invention, the desirable target concentration is between 20 to 30 ng/ml. The precise loading dose and the daily replacement dose can be calculated either as a mean dose necessary for obtaining the desirable mean serum concentration in the majority of the subjects or can be calculated based on the personalized baseline data of the subject and the desirable concentration. The bolus dose can be calculated based on the pool size of 25OHD (18 liters for a normal sized adult) multiplied by the difference between desirable serum concentration (20 or 30 ng/ml) and the mean or individual baseline serum concentration. In particular embodiments, the loading dose is at least 200 µg 25OHD. For instance, to calculate the bolus dose of 25OHD to increase serum 25OHD from a mean baseline serum 25OHD concentration of 8 ng/ml and to achieve the target concentration of at least 20 ng/ml (as recommended by IOM and other guidelines) one determines the difference between the baseline and target concentration and multiplies this by the loading volume. In this situation a bolus dose of (20-8) x 18 liter = 216 ug is required. In the more extreme baseline situation of 4 ng/ml (= about the lowest concentration found in clinical studies) and to achieve a target concentration of 20 ng/ml one needs to determine the difference in concentration (20-4 ug) multiplied by the distribution volume of 18 liters = 288 ug of 25OHD)). If the physician aims to achieve target concentrations of 30 ng/ml, as recommended by some experts, following the Endocrine society guidelines, then the loading dose of 25OHD has to be calculated based thereon. For a baseline of 8 ng/ml to increase to 30 ng/ml, the dose should be (30-8) x 18 = 396 ug. For a patient with a very low starting concentration of 4 ng/ml the bolus dose should be (30-4) x 18 = 468 ug. From these examples, it can be established that for critically ill patients, the loading volume is at least 200µg, preferably at least 225µg, at least 250µg, or, where there are indications of very low serum 25OHD levels or where a target concentration of 30ng/ml is envisaged, this may be at least 300µg.

In particular embodiments, the treatment encompasses administering a bolus dosage of at least 200µg and thereafter administering a daily restoration dosage of at least 25 µg 25OHD/day to said patient for at least 2 consecutive days. In further embodiments, the methods comprise determining the optimal bolus dosage and/or determining the optimal daily restoration dosage.

The methods of the invention are based on the observation that the method as described herein which comprises the administration of at least 25 µg 25OH/day ensures a restoration of the vitamin D deficiency in said patient. This dose is at least 5 times higher than could be predicted based on the prior art.

As detailed above, the calculation of the daily dosage can be based on the amount of 25OHD that has been determined to ensure a serum increase serum 25OHD in an average adult. In particular embodiments however, the calculation of the daily dosage takes into account average weight and height of an adult patient. In particular embodiments, the method comprises administering 30µg/day, 35, 40, 45, 50, 55 or even 60µg/day or more or any value in between. The methods of the invention typically comprise administering said at least 25µg/day for at least two days, and frequently will comprise administering said at least 25µg/day for 3, 4, 5, 6, 7, 8, 9, 10, 11, 12, 13, 14 days or longer depending on the health status of the patient.

In particular embodiments, the treatment considered herein comprises administering at least 25 µg 25OHD/day, such as at least 30 µg 25OHD/day or at least 35 µg 25OHD/day, for at least 5, at least 7, or at least 10 consecutive days to said patient. Preferably, the administration of at least 25 µg 25OHD/day, such as at least 30 µg 25OHD/day or at least 35 µg 25OHD/day is performed during the whole ICU period, i.e. until the patient is no longer critically ill.

In particular embodiments, the treatment considered herein is performed until the level of serum 25OHD in a plasma sample of said patient is restored to the appropriate level, such as to at least 10ng/ml, at least 20 ng or 30 or more nanograms of 25OHD/ml. In particular embodiments, the treatment considered herein is performed until the level of serum 25OHD in a plasma sample of said patient is measured to be at the appropriate level. Preferably, the treatment considered herein is performed until the level of serum 25OHD in a plasma sample of said patient has reached at least 20 ng (as recommended by most guidelines) or at least 30 ng/ml (as recommended by inter alia the Endocrine society and) for at least 2 days, such as at least 7 days or more.

In particular embodiments, the treatment considered herein comprises determining the vitamin D status as defined by the serum 25OHD level of said patient. For instance, the method may comprise determining whether the 25OHD level of said patient has reached the desired level. Methods for determining the level of 25OHD in a serum sample are known in the art and include antibody-based methods such as RIA, CLIA or ELISA, or direct methods such as LC-MS/ MS or HPLC. In particular embodiments, the the serum 25OHD level of said patient is measured by mass spectrometry. In line with recent guidelines, the method used to measure serum 25OHD should not only be precise but also reflect the accurate level by using methods that are validated in comparison with the National Institute of Standard and Technology (NIST-US or similar institutions) (ref Sempos Br J Pharm 2018). The level of 25OHD in the methods of the invention can be measured according to a regular or varying schedule, such as daily or intermittently, varying from once every 2, 3, 4, 5, 6, 7 or 14 days. In particular embodiments, the level is measured once every two to seven days.

In particular embodiments, the methods comprise determining the vitamin D status, in particular the serum 25OHD level and adjusting the dosage of the 25OHD accordingly. More particularly, it can be envisaged that, if in a particular patient, the serum level of 25OHD is not restored within 2, 3, 4, 5, 6 or 7 days, that the daily dosage of 25OHD is increased by 1, 2, 3, 4, 5, 6, 7, 8, 9 to 10µg. In particular embodiments, the the serum 25OHD level of said patient is measured by mass spectrometry. The level of 25OHD in the methods of the invention can be measured daily, once every two days, or once every 3, 4, 5, 6, or 7 days. Preferably the 25OHd level is measured between once every 2 to 7 or 14 days.

In the methods of the invention, the method of administration is not critical. However, for the administration to patients which are critically ill, intravenous administration is typically preferred. Intravenous administration can be by injection (i.e. with a syringe) or by infusion (which can be based on gravity or pump-driven). Typically, the administration is by intravenous infusion. This typically implies that a dosage of 1ml of a 25µl/ml 25OHD solution is added to the IV infusion line.

Alternative forms of administration are known in the art such as oral, anal, transdermal, and other forms of administration. In patients where the critically ill patients do not have any gastrointestinal issues and are capable of taking oral medication, oral administration may be preferred.

The nutritional treatment of a critically ill patient typically also comprises administering to a daily dosage of cholecalciferol supplement. In particular embodiments, such daily dosage will be of of +/-200 (5µg) IU vitamin D. The vitamin D supplement can be administered in any way but is similarly preferably administered intravenously such as dissolved in 10ml of parental nutrition (PN) solution. Examples of suitable parental nutrition solutions include but are not limited to Vitalipid®, Cemevit®, A.S.P.E.N. etc In particular embodiments, the methods also include this in the treatment of said patient. However, as detailed herein, this is not critical to the invention as it is demonstrated herein that this dose of simple vitamin D does not help correct the vitamin D status of such patients.

As detailed above, the invention also provides methods for determining the optimal dosage of 25OHD for administration to a critically ill patient, in order to obtain a target 25OHD concentration.

The present application also includes a pharmaceutical composition comprising formulations, such as intravenous or oral formulations, of 25OHD. In particular, the bolus and/or daily dose of 25OHD is included in an isotonic sterile formulation of 25OHD. Suitable carriers for intravenous administration include physiological saline or phosphate buffered saline (PBS), and solutions containing solubilizing agents, such as glucose, polyethylene glycol, and polypropylene glycol and mixtures thereof. Suitable carriers for oral administration are known in the art. In particular embodiments, absorption enhancers and/or enzymatic inhibitors are combined therewith. Accordingly, in particular embodiments, the application provides pharmaceutical compositions comprising between 25 and 60µg 25OHD. In further particular embodiments, the invention provides pharmaceutical compositions comprising between 25 and 60 µg/ml OHD in a suitable solvent. Suitable solvents for IV administration include, but are not limited to, ethanol. However, 25OHD can also be dissolved in other solvents such as water or lipids. For oral administration other formulations can also be envisaged. Such compositions can be provided in sterile vials. The application further comprises sets of vials comprising between 25 and 60 µg 25OHD, wherein said sets comprise at least 2, preferably 3, 4, 5, 6, 7 or more vials, suitable for administration to a patient on consecutive days according to the methods of the present invention. In particular embodiments the invention provides combinations of a first composition comprising between 25 and 60 µg 25OHD and a second composition comprising at least 200µg, such as between 200-300µg 25OHD. The former composition can be formulated as comprising between 25 and 60µg 25OHD/ml and/or the latter composition can be formulated as comprising at least 200µg 25OHD/ml. In particular embodiments, said sets comprise at least 2 compositions comprising between 25 and 60 µg 25OHD, such as between 25 and 60µg 25OHD/ml.

The preparation of a composition comprising 25OHD, particularly comprising at least 25 µg 25OHD, or higher doses (as described above) suitable for administration according to the present invention, is within the ordinary skill of the skilled person. In certain embodiments, the composition is for IV administration and such compositions are prepared by dissolving the desired amount of 25OHD in absolute ethanol, propylene glycol or other suitable solvents, and, particularly, combining the resulting solutions with surfactants, salts and preservatives in appropriate volumes of water for injection. Such formulations can be administered directly from syringes or other vials. The composition can be administered as a single administration shot. The dosage forms may also contain adjuvants, such as preserving or stabilizing adjuvants. They may also contain other therapeutically valuable substances. For instance, the treatment as considered herein may comprise the intravenous administration of 25OHD as described above, in combination with cholecalciferol, ergocalciferol, active Vitamin D sterols, glycemic and hypertension control agents, and/or various antineoplastic agents.

In particular embodiments, the pharmaceutical composition is packaged in a vial, an ampoule or a prefilled syringe.

The present invention is now further illustrated via the following non-limiting example.

### EXAMPLES

### Example 1: Treatment of critically ill patients with 25OHD

### Materials and methods

### Study design

The study was performed in accordance with the 1964 Declaration of Helsinki and its later amendments. The protocol and consent forms were approved by the institutional review board of the University Hospitals Leuven (ML2462). The study was registered at www.controlled-trials.com (ISRCTN24385496). Patients with an anticipated stay in the intensive care unit (ICU) of more than 10 days were eligible for inclusion in the study. Patients younger than 18 years, those suffering from chronic bone or kidney disease, and those who were treated with glucocorticoids before ICU admission were excluded. Patients were compared with healthy controls matched for age, gender and body mass index, of whom blood was sampled during summer time.

Upon ICU admission, patients were randomly allocated to either a daily IV injection of placebo (NaCl 0.9%) or to an IV loading dose of 200 µg 25OHD, followed by an IV replacement dose of 15 µg 25OHD per day, from ICU admission onward and continued for 10 days. Patients from both groups also received the usual vitamin D supplement, which consisted of a daily intravenous cholecalciferol supplement of +/-200 IU (5 µg, as part of 10 ml of Cernevit® (Clinitec-Baxter, Brussels, Belgium). 25OHD was obtained from Solvay Pharmaceuticals (Brussels, Belgium) and dissolved in ethanol by the hospital pharmacy under laminar flow conditions in glass vials, containing 200 µg/ml per vial for the loading dose and 15 µg/ml per vial for the replacement dose. A purity control was performed on the prepared samples using HPLC. Similarly, placebo vials were also prepared by the hospital pharmacy (1 ml NaCl 0.9% per vial). All vials were blinded by the hospital pharmacy.

### Endpoints

The effect of IV 25OHD supplementation on vitamin D status (25OHD, 1,25(OH)2D, 24,25(OH)2D) was studied. Clinical outcome parameters (organ dysfunction (sequential organ failure assessment (SOFA) score, days on mechanical ventilation, need for renal replacement therapy), prolonged (>3 days) antibiotic therapy, ICU length of stay and mortality) were registered as safety endpoints. All patients were followed up and monitored for possible adverse events beyond the intervention period of 10 days until discharge or death. Analysts and investigators were blinded for treatment allocation.

### Blood analyses

Every day at 06.00 a.m., from ICU admission until ICU discharge or death, undiluted blood samples were taken through an arterial line that had been placed for clinical purposes. Serum and plasma samples were frozen and subsequently stored at -20°C until assay. Concentrations of 25OHD, 1,25(OH)2D, and 24,25(OH)2D were measured in serum.

25OHD was measured by competitive binding assay; 1,25(OH)2D by radioimmunoassay (RIA), and DBP by radial immunodiffusion, as previously reported by Bouillon et al (J Clin Invest 1981 67 589-596). A subset of the original 25OHD measurements were subsequently validated by re-analyses using LC-MS/MS, currently considered the gold standard. To this end, 58 samples from patients (treated with 25OHD or placebo) and controls were selected at random covering the full range of 25OHD serum concentrations (3.7-70.7 ng/ml). Briefly, serum proteins were precipitated using zinc sulphate solution, spiked with *d6*-25OHD₃ as internal standard. After mixing and centrifugation, the supernatant was transferred to the liquid chromatography autosampler. We used a ScieX 5500 QTRAP mass spectrometer connected with a Shimadzu chromatographic system. The method consisted of an online clean-up step using a Strata C8 extraction cartridge. After loading and cleaning, the analytes were eluted by back-flushing. A Kinetex F5 column was used as analytical column. Atmospheric-pressure chemical ionization (APCI) positive mode was used. Mass transitions of 383>211, 295>211 and 389>211 were used for 25OHD3, 25OHD2 and the internal standard, respectively. Within-run and between-run CVs at clinically relevant concentrations varied between 6.3-7.4% and 6.9-8.8% respectively. Overall, LC-MS/MS measurements revealed lower values (p<0.0001), but the agreement with RIA was fair (y = 1.36 x - 0.67; r² = 0.83 with linear regression). Serum 25OHD was also measured in 20 DEQAS samples with NIST validated concentrations. The regression line between the DEQAS values and the LC-MS/MS 25(OH)D was y = 1.0025 x + 1.76 (r² = 0.96), thus the LC-MS/MS must be considered as accurate. All original 25OHD values were subsequently recalibrated with use of the obtained regression equation Therefore, the corrected 25OHD concentrations can be considered as traceable to the NIST, Ghent and CDC reference methods.

Serum concentrations of 24,25(OH)2D were measured by LC-MS/MS as described in Cools et al (Bone 2015 81: 89-96).

### Statistical analysis

StatView 5.0.1 (SAS Institute Inc., Cary, NC) and JMP13 (SAS) were used for the statistical analyses. Data are presented as mean and standard deviation, median and interquartile range, or proportion and percentages, as appropriate. Variables with a normal distribution were analyzed with Student's t-test, variables with a non-normal distribution with Mann-Whitney U, and proportions with the chi-square test. Fisher's exact test was used to compare admission diagnoses. Repeated-measures ANOVA was used to compare the time courses in different groups. Two-sided p values below 0.05 were considered significant.

### Results

### Patients

Thirty-four patients initiated the study, of which only 24 required intensive care for more than 10 days. Ten patients were thus eventually excluded from the study because of early discharge from ICU. Nevertheless, these patients were followed for adverse events and safety endpoints, as they received part of the prescribed study medication. Of the 24 remaining patients, 11 had been randomized to the 25OHD group and 13 patients to the placebo group. Baseline demographic and clinical data of the patients demonstrated that the patients were comparable for age, gender, body mass index, severity of illness (first 24 h APACHE-II score), and markers of kidney function and inflammation. Likewise, reasons for admission to ICU were comparable for the 2 groups.

### Impact of critical illness on markers of vitamin D metabolism

Twenty-four healthy controls were matched for age, gender and BMI to the critically ill patients. Upon admission, critically ill patients presented with 25OHD, 1,25(OH)2D and 24,25(OH)2D concentrations that were significantly lower than those in healthy controls.

### Effect of IV 25OHD supplementation on serum concentrations of 25OHD, 1,25(OH)2D and 24,25(OH)2D

Upon ICU admission, markers of vitamin D status were comparable for patients receiving placebo or 25OHD, except for PTH. Likewise, baseline concentrations of selected markers of bone metabolism (sALP, osteocalcin, FGF23 and β-crosslaps), and CRP, were comparable in both treatment arms.

Following the intravenous loading dose of 200 µg 25OHD, 25OHD serum concentrations instantly rose but did not reach the 20 ng/ml target (Figure 1A). The increase in serum 25OHD of 11 ng/ml after this bolus allows to estimate an approximative distribution volume of 18 liters. 25OHD remained elevated throughout the 10-day period during which patients were treated with a replacement dose of 25OHD as compared with placebo (Figure 1). Nevertheless, these 25OHD concentrations remained substantially lower than those observed in summer in the matched healthy subjects.

Despite the persistent rise in the 25OHD substrate availability, the treatment only transiently increased the concentrations of 1,25(OH)2D, which also remained lower than those in healthy controls at all time points (Figure 1B). DBP concentrations, lowered upon admission, were restored after 2 days to values comparable with those for healthy controls, but were unaffected by the treatment (Figure 1c). Molar ratios of 1,25(OH)2D to DBP steadily decreased further over time in ICU. These molar ratios only transiently rose upon 25OHD supplementation, but remained significantly lower than controls, and were over time, further unaffected by treatment.

As the supplemented 25OHD appeared not to be converted to its active metabolite 1,25(OH)2D, we explored an alternative metabolic pathway, namely the conversion to the inactive metabolite 24,25(OH)2D. For all patients and controls, serum 24,25(OH)2D concentrations were strongly correlated with 25OHD concentrations. At baseline, 24,25(OH)2D concentrations were significantly lower in patients as compared to the healthy controls. Patients had a lower 24,25(OH)2D concentration (7.0%) relative to their 25OHD concentration in comparison with controls (11.3%) (p<0.0001). Supplementation with 25OHD resulted only in a very small increase in 24,25(OH)2D concentrations, which, however, never reached the healthy control values (less than 1 ng/ml vs about 3 ng/ml for healthy subjects).

The relative ratio of serum 25OHD to 24,25(OH)2D, which is a marker of vitamin D catabolism, was also calculated. At admission, patients had a higher 25OHD to 24,25(OH)2D ratio than healthy controls (14 [10.5-18.5] in patients versus 8.5 [7-9.8] in healthy volunteers, p <0.0001). 25OHD supplementation did not alter these ratios, which did not differ between the 25OHD treated patients and the placebo group at any investigated time point during the study period, (14 [10-22] in the calcifediol group versus 14 [10-18] in the placebo group at admission, p=0.9; 19 [16-25] versus 19 [14-21] at day 5, p=0.7; and 15 [12-19] versus 15 [13-35] at day 10, p=0.9).

### Conclusion

The results of the present randomized, placebo-controlled, pilot study confirm previous findings showing that critically ill patients have low concentrations of vitamin D metabolites (with baseline of about 8 +/- 4 ng/ml). Low vitamin D status with reduced concentrations of the circulating precursor 25OHD and the active 1,25(OH)2D is prevalent during prolonged critical illness, starting upon admission to the ICU. ICU patients are particularly prone to develop low 25OHD concentrations, which can be attributed to insufficient vitamin replacement, lack of UV-B exposure and vitamin D wasting. Moreover, critical illness-related organ dysfunction potentially affects vitamin D metabolism, as hepatic and renal dysfunction can impair the conversion of vitamin D (calciferol) to 25OHD and 1,25(OH)2D, respectively.

The present study clearly shows that intravenous 25OHD can immediately correct the vitamin D status (as evaluated by serum 25OHD). With respect to the dosage, the present study shows that a daily supplement of 15 µg of 25OHD increased the 25OHD levels only by about 8 ng/ml. The 25OHD levels in the subgroup receiving the active treatment increased from 9.2 by 6.8 ng/ml. While the serum levels of 25OHD could be maintained at a fairly stable rate, the mean level was only of about 16 ng/ml, which is still well below the minimal desirable threshold of 20 ng/ml. Thus, this implies that 1µg/ml ensured an increase of 0.45ng/ml. This indicates that the catabolism of 25OHD in ICU patients is at least 3-fold higher than in normal subjects of the same age. This study also shows that the poor vitamin D status of trauma/ICU patients can only be restored by supra-physiologic doses of 25OHD.

It can be derived that, in critically ill patients, one µg of 25OHD increases serum 25OHD level by about 0.45 ng/ml. Stated differently, for raising the 25OHD levels in ICU patients from (mean) 8 ng/ml to minimal 20 ng/ml, would thus require a daily IV dose of about 26.7 µg 25OHD. For raising the 25OHD levels in ICU patients from percentile 10 (4 ng/ml) to 20 ng/ml would require about 35 µg 25OHD/day (by intravenous administration). This dose is about 5-7 fold higher than a skilled person could have predicted.

### Example 2: Further trial of the treatment of critically ill patients with 25OHD

The study design and materials are similar to the one described in Example 1, the difference being that the patients are administered higher bolus and daily replacement doses of 25OHD for at least two days after the bolus administration and preferably during the hospital stay of such patients .

### Materials and methods

### Study design

The study is performed in accordance with the 1964 Declaration of Helsinki and its later amendments. Patients with an anticipated stay in the intensive care unit (ICU) of more than 10 days are considered eligible for inclusion in the study. Patients younger than 18 years, those suffering from chronic bone or kidney disease, and those who were treated with glucocorticoids before ICU admission are excluded. Patients are compared with healthy controls matched for age, gender and body mass index, of whom blood was sampled during summer time.

Upon ICU admission, patients are randomly allocated to either a daily IV injection of placebo (NaCl 0.9%) or to an IV loading dose of 200 µg 25OHD, followed by an IV replacement dose of 25 µg 25OHD per day, from ICU admission onward and continued for 10 days. Patients from both groups also receive the required vitamin D supplement, which consisted of a daily intravenous cholecalciferol supplement of +/-200 IU (5 µg, as part of 10 ml of Cernevit® (Clinitec-Baxter, Brussels, Belgium). 25OHD is obtained as above and dissolved in ethanol by the hospital pharmacy under laminar flow conditions in glass vials, containing 225 µg/ml per vial for the loading dose and 25 µg/ml per vial for the daily restoration dose. A purity control is performed on the prepared samples using HPLC. Similarly, placebo vials are also prepared by the hospital pharmacy (1 ml NaCl 0.9% per vial). All vials are blinded by the hospital pharmacy.

### Endpoints

The effect of IV 25OHD supplementation on vitamin D status (25OHD) is studied. Clinical outcome parameters (organ dysfunction (sequential organ failure assessment (SOFA) score, days on mechanical ventilation, need for renal replacement therapy), prolonged (>3 days) antibiotic therapy, ICU length of stay and mortality) are registered as safety endpoints. All patients are followed up and monitored for possible adverse events beyond the intervention period of 10 days until discharge or death. Analysts and investigators are blinded for treatment allocation.

### Blood analyses

Every day at 06.00 a.m., from ICU admission until ICU discharge or death, undiluted blood samples are taken through an arterial line that is present for clinical purposes. Serum and plasma samples are frozen and subsequently stored at -20°C until assay. Concentrations of 25OHD are measured in serum.

25OHD is measured by a validated assay and preferably by using LC-MS/MS (Sempos et al, 2018, Br J Clin Pharmacol. 2018 Oct;84(10):2194-2207. doi: 10.1111/bcp.13652)

### Statistical analysis

StatView 5.0.1 (SAS Institute Inc., Cary, NC) and JMP13 (SAS) is used for the statistical analyses. Data are presented as mean and standard deviation, median and interquartile range, or proportion and percentages, as appropriate. Variables with a normal distribution are analyzed with Student's t-test, variables with a non-normal distribution with Mann-Whitney U, and proportions with the chi-square test. Fisher's exact test is used to compare admission diagnoses. Repeated-measures ANOVA is used to compare the time courses in different groups. Two-sided p values below 0.05 were considered significant.

### Results

### Patients

Patients who are expected not to need intensive care treatment for at least 10 days are excluded from the study. The remaining patients are distributed randomly into the 25OHD group and the placebo group. Baseline demographic and clinical data of the patients demonstrate that the patients are comparable for age, gender, body mass index, severity of illness (first 24 h APACHE-II score), and markers of kidney function and inflammation. Likewise, reasons for admission to ICU are comparable for the 2 groups.

### Effect of IV 25OHD supplementation on serum concentrations of 25OHD

The majority of the patients in the 25OHD group are found to reach the 20 µg/ml target concentration of 25OHD. A positive effects is observed on the safety endpoints.

## Claims

1. A pharmaceutical composition comprising 25OHD for use in the treatment of vitamin D deficiency in a critically ill patient, wherein said treatment comprises administering the equivalent of a daily dosage of at least 25 µg 25OHD/day to said patient over a period of at least two days.

2. The composition according to claim 1, wherein said administration is an intravenous or an oral administration.

3. The composition according to claim 1 or 2, wherein the method comprises administering a bolus of 25OHD on day 0 prior to administering a daily dosage of at least 25 µg 25OHD/day as of day 1, for at least 2 consecutive days.

4. The composition according to claim 3, wherein the bolus of 25OHD is at least 200µg.

5. The composition according to claim 4, wherein said bolus is determined based on the distribution volume and the target serum concentration.

6. The composition according to any one of claims 1 to 5, wherein the starting 25OHD serum concentration of said patient is determined prior to treatment and said daily dosage is determined based on the difference between a target concentration and said starting concentration.

7. The composition according to any one of claims 1 to 6, wherein said treatment comprises administering said dosage of at least 25µg 25OHD /day until the level of serum 25OHD in a plasma sample of said patient is restored to at least 20 ng/ml or to at least 30 ng/ml.

8. The composition according to any one of claims 1 to 7, wherein said treatment comprises administering a dosage of at least 25 µg 25OHD/day for at least 5 or at least 7 consecutive days to said patient.

9. The composition according to any one of claims 1 to 8, wherein said treatment comprises, determining the serum 25OHD level of said patient after two days, and adjusting the dosage of said 25OHD accordingly.

10. A pharmaceutical composition comprising between 25 and 60 µg 25OHD.

11. A kit comprising a pharmaceutical composition comprising a first vial comprising between 25 and 60 µg/ml 25OHD in ethanol and a second vial comprising between 200-300µg/ml of 25OHD.

12. A method for determining the bolus dosage for administering to a patient or patient group for treating vitamin D deficiency, said method comprising
(a) determining the serum 25OHD concentration of said patient or patient group,
(b) determining the target 25OHD concentration of said patient or patient group, and
(c) determining the bolus dosage for administration to said patient or patient group based on the distribution volume in said patient.

13. The method of claim 12, wherein said patient is a critically ill patient and said distribution volume is 18 L.
